# FASCICULE DE BREVET EUROPEEN

(11) **EP 3 154 465 B1**
(45) Date de publication et mention de la délivrance du brevet: **21.06.2023**
(21) Numéro de dépôt: 15732873.3
(22) Date de dépôt: 12.06.2015
(51) Int. Cl.: A61B 46/10, A61M 25/01, A61B 34/30

(54) **MODULE ROBOTISE D'ENTRAÎNEMENT D'ORGANE MÉDICAL SOUPLE ALLONGÉ**
ROBOTISIERTES MODUL ZUR FÜHRUNG EINER LANGGESTRECKTEN FLEXIBLEN MEDIZINISCHEN VORRICHTUNG
ROBOTIZED MODULE FOR GUIDING AN ELONGATE FLEXIBLE MEDICAL DEVICE

(30) Priorité: 12.06.2014 FR 1455330
(43) Date de publication de la demande: 19.04.2017
(73) Titulaire: Robocath, 76000 Rouen (FR)
(72) Inventeur: DEBOEUF, Sébastien, F-76240 Bonsecours (FR); DESTREBECQ, Fabien, F-27520 Bourgtheroulde (FR); FOURNIER, Bruno, F-41100 Saint Ouen (FR); BENCTEUX, Philippe, F-76160 Saint Martin du Vivier (FR)
(74) Mandataire: Plasseraud IP
(86) Numéro de dépôt international: PCT/FR2015/051562
(87) Numéro de publication internationale: WO 2015/189529

(56) Documents cités:
- EP-A1- 2 567 670
- WO-A2-2014/005689
- US-A1- 2010 170 519

## Description

La présente invention est relative aux modules robotisés d'entraînement d'organes médicaux souples allongés.

L'insertion manuelle d'un cathéter ou d'un guide dans un patient est un acte chirurgical relativement classique. Toutefois, cet acte étant monitoré sous rayons X, le chirurgien en charge de cet acte est soumis à une irradiation conséquente s'il réalise une telle opération sur de nombreux patients.

Afin de réduire les risques pour le chirurgien, on tente de robotiser une telle insertion. Cette robotisation est complexe, car la préhension du cathéter est difficile. Celui-ci baigne en effet dans du liquide de conservation et doit rester stérile. Par ailleurs, on veut pouvoir commander des mouvements de translation et de rotation alternatifs et/ou simultanés du cathéter. La fiabilité de ces systèmes robotisés est un critère déterminant.

Récemment, il a été proposé dans US 7,927,310 un système d'entraînement gérant à la fois la translation et la rotation du cathéter. Le cathéter est maintenu sur une plaquette rotative par rapport à une base pour l'entraînement en rotation. La plaquette elle-même comporte un mécanisme d'entraînement en translation. De plus, on fait appel à des moteurs déportés, à demeure sur le bâti, et à des systèmes de transfert du mouvement jusqu'au cathéter. En effet, on préfère ne pas avoir les moteurs embarqués, pour des raisons d'alimentation en puissance, d'encombrement et de stérilité. Les documents EP 2 567 670 A1, WO 2014/005689 A2, et US 2010/170519 A1 sont également des documents connus de l'état de la technique.

Bien que cette configuration apporte toute satisfaction, on cherche toujours à simplifier ce genre de mécanismes en vue notamment d'en augmenter la fiabilité.

A cet effet, selon l'invention, on prévoit un module robotisé d'entraînement d'organe médical souple allongé comprenant :
- une base,
- une paire d'organes d'entraînement présentant chacun une surface d'entraînement, la paire d'organes d'entraînement étant plaçable dans une configuration d'entraînement dans laquelle les surfaces d'entraînement des organes d'entraînement de la paire d'organes d'entraînement sont en prise avec l'organe médical souple allongé à entraîner et disposés de part et d'autre de celui-ci,
la paire d'organes d'entraînement étant montée mobile par rapport à la base selon un degré de liberté entre une première et une deuxième positions,
- un organe de commande adapté pour commander un déplacement par rapport à la base des organes d'entraînement de la paire d'organes d'entraînement en configuration d'entraînement de la première à la deuxième position, entraînant ainsi l'organe médical souple allongé par rapport à la base,
- une barrière stérile consommable à usage unique comprenant un pion d'accrochage pour chaque organe d'entraînement, et des portions souples entre deux pions d'accrochage voisins, les portions souples étant solidaires des pions d'accrochage, chaque organe d'entraînement étant attaché à un pion d'accrochage, les surfaces d'entraînement des pions d'accrochage étant destinées à venir en contact avec l'organe médical souple allongé.

Grâce à ces dispositions, l'entraînement de l'organe médical souple allongé, même sur des trajectoires longues et complexes, peut être mis en oeuvre de manière stérile.

Dans des modes de réalisation préférés de l'invention, on peut éventuellement avoir recours en outre à l'une et/ou à l'autre des dispositions suivantes :
- la paire d'organes d'entraînement est plaçable alternativement dans la configuration d'entraînement et dans une configuration libre dans laquelle la surface d'entraînement des organes d'entraînement de la paire d'organes d'entraînement n'est pas en prise avec l'organe médical souple allongé,

l'organe de commande est adapté pour commander de manière répétée cyclique le déplacement par rapport à la base des organes d'entraînement en configuration d'entraînement de la première à la deuxième position, et un déplacement par rapport à la base des organes d'entraînement de la paire d'organes d'entraînement en configuration libre de la deuxième à la première position sans entraîner l'organe médical souple allongé par rapport à la base ;
   - la base est une première base, la paire d'organes d'entraînement est une première paire d'organes d'entraînement, le module robotisé comprenant en outre :
      · une deuxième base,
      · une deuxième paire d'organes d'entraînement présentant chacun une surface d'entraînement, la deuxième paire d'organes d'entraînement étant plaçable dans une configuration d'entraînement dans laquelle les surfaces d'entraînement des organes d'entraînement de la deuxième paire d'organes d'entraînement sont en prise avec l'organe médical souple allongé à entraîner et disposés de part et d'autre de celui-ci,,
la deuxième paire d'organes d'entraînement étant montée mobile par rapport à la deuxième base selon un degré de liberté entre une première et une deuxième positions,
   · l'organe de commande étant adapté en outre pour commander un déplacement par rapport à la base des organes d'entraînement de la deuxième paire d'organes d'entraînement en configuration d'entraînement de la première à la deuxième position, entraînant ainsi l'organe médical souple allongé par rapport à la deuxième base ;

   - la deuxième paire d'organes d'entraînement est plaçable alternativement dans la configuration d'entraînement et dans une configuration libre dans laquelle la surface d'entraînement des organes d'entraînement de la deuxième paire d'organes d'entraînement n'est pas en prise avec l'organe médical souple allongé,
l'organe de commande est adapté pour commander de manière répétée cyclique le déplacement par rapport à la deuxième base des organes d'entraînement en configuration d'entraînement de la première à la deuxième position, et un déplacement par rapport à la deuxième base des organes d'entraînement de la deuxième paire d'organes d'entraînement en configuration libre de la deuxième à la première position sans entraîner l'organe médical souple allongé par rapport à la deuxième base ;
   - la première base et la deuxième base sont solidaires ou communes ;
   - la barrière stérile consommable à usage unique comprend un pion d'accrochage pour chaque organe d'entraînement de la deuxième paire, et des portions souples entre deux pions d'accrochage voisins, les portions souples étant solidaires des pions d'accrochage, chaque organe d'entraînement de la deuxième paire étant attaché à un pion d'accrochage, les surfaces d'entraînement des pions d'accrochage étant destinées à venir en contact avec l'organe médical souple allongé ;
   - la barrière stérile comprend en outre un film continu consommable à usage unique comprenant une portion d'attachement pour chaque organe d'entraînement, et des portions souples entre deux portions d'attachement voisines, chaque organe d'entraînement étant attaché à une portion d'attachement du film continu, les surfaces d'entraînement étant en prise avec l'organe médical souple allongé par l'intermédiaire du film continu ;
   - la barrière stérile comprend également un carter rigide, chaque pion d'accrochage étant assemblé au carter rigide par l'intermédiaire desdites portions souples ;
   - le carter rigide comprend une pluralité de fenêtres fermées chacune de manière étanche par une portion souple ;
   - le pion d'accrochage est assemblé à l'organe d'entraînement par l'intermédiaire de zones frangibles reliant la surface d'entraînement, en contact avec l'organe médical souple allongé, à la portion d'assemblage à l'organe d'entraînement ;
   - la barrière stérile sépare l'espace en deux sous-espaces : un sous-espace stérile comprenant l'organe médical souple allongé, et un sous-espace pas nécessairement stérile dans lequel sont disposés les organes d'entraînement ;
   - le module robotisé d'entraînement comprend un boîtier fermé englobant les organes d'entraînement et, le cas échéant, la base, et comportant une lumière amont et une lumière aval à travers lesquelles s'étend l'organe médical souple allongé ;
   - le boîtier comprend également un organe de liaison de tout organe d'entraînement à un moteur externe au boîtier ;
   - le boîtier comporte un réceptacle et un couvercle mobile par rapport au réceptacle entre une configuration fermée où un accès par un opérateur à l'organe médical souple allongé à l'intérieur du boîtier est interdit, et une configuration ouverte où un accès par un opérateur à l'organe médical souple allongé à l'intérieur du boîtier est possible ;
   - une portion finale de la course du couvercle depuis sa configuration ouverte à sa configuration fermée est un mouvement de translation ;
   - en configuration fermée, au voisinage des organes d'entraînement, un volume libre important est disponible entre le couvercle et le réceptacle, et en dehors de ce voisinage, un volume libre réduit est disponible entre le couvercle et le réceptacle, y permettant un guidage de l'organe médical souple allongé ;
   - le boîtier comporte une portion de base à demeure portant les organes d'entraînement, et une cassette consommable à usage unique assemblable/désassemblable de la portion de base, la cassette comprenant au moins la barrière stérile ;
   - le réceptacle comprend la portion de base et la barrière stérile ;
   - chaque pion d'accrochage est détachable d'un organe d'entraînement respectif ;
   - le module robotisé comprend en outre un véhicule porteur de pions d'accrochage, rigide, chaque pion d'accrochage étant assemblé de manière provisoire au véhicule ;
   - le véhicule comprend en outre une portion de détrompage empêchant son assemblage selon une orientation non souhaitée ;
   - le véhicule est désassemblable de la barrière stérile ;
   - chaque pion d'accrochage comporte une paroi plane comportant une face avant, et comprenant un système barrière de limitation du déplacement de l'organe médical souple allongé, maintenant l'organe médical souple allongé en regard de la face avant ;
   - chaque barrière du système barrière de limitation comprend une alternance de creux et de saillies.

Selon un autre aspect, l'invention se rapporte à un robot d'artériographie comprenant un récipient, un organe médical souple allongé au moins partiellement contenu dans le récipient, un module robotisé d'entraînement fixé au récipient, et adapté pour l'entraînement de l'organe médical souple allongé hors du récipient.

Selon un autre aspect, un exemple se rapporte à une cassette comprenant une barrière stérile comprenant des pions d'accrochage adaptés pour être attachés chacun à un organe d'entraînement d'un organe médical souple allongé, et des portions souples entre deux pions d'accrochage voisins, les portions souples étant solidaires des pions d'accrochage, les surfaces d'entraînement des pions d'accrochage étant destinées à venir en contact avec l'organe médical souple allongé.

D'autres caractéristiques et avantages de l'invention apparaîtront au cours de la description suivante d'une de ses formes de réalisation, donnée à titre d'exemple non limitatif, en regard des dessins joints.

Sur les dessins :
- la figure 1a est une vue schématique de côté d'une installation d'artériographie robotisée.
- la figure 1b est une vue de dessus d'une partie de la figure 1a,
- la figure 2 est une vue schématique de dessus d'un robot utilisé dans l'installation des figures 1a et 1b,
- les figures 3a-3c sont des schémas illustratifs des modes de déplacement des organes à entraîner,
- la figure 4 est une vue schématique en perspective d'une portion d'un module d'entraînement en configuration libre,
- la figure 5a est une vue plane de face d'un module selon un mode de réalisation,
- la figure 5b est une vue plane de dessus du module de la figure 5a,
- la figure 6 est une vue similaire à la figure 5a, sans transparence, en configuration ouverte du boîtier,
- la figure 7 est une vue schématique de face d'un organe d'entrainement,
- la figure 8 est une vue en perspective de dessus d'un exemple de réalisation de cassette (sans couvercle),
- la figure 9 est une vue en coupe selon la ligne IX-IX de la figure 8,
- la figure 10 est une vue en perspective d'une cassette avec couvercle,
- les figures 11a et 11b sont des vues en perspective de l'arrière d'un pion d'accrochage et d'un embout, repectivement
- la figure 12 est une vue similaire à la figure 11a où l'embout est assemblé au pion d'accrochage,
- la figure 13 est une vue en perspective de dessus de la cassette intégrant un porte-pions,
- la figure 14 est une vue en coupe selon la coupe IX-IX du porte-pions,
- les figures 15a-15f sont des vues en coupe de côté qui représentent diverses étapes d'assemblage du pion à l'embout selon une variante de réalisation,
- la figure 16 est une vue en perspective de l'arrière qui représente une variante de réalisation de pion,
- la figure 17 est une vue en perspective de l'arrière représentant une variante de réalisation de pion d'accrochage.

Sur les différentes figures, les mêmes références désignent des éléments identiques ou similaires.

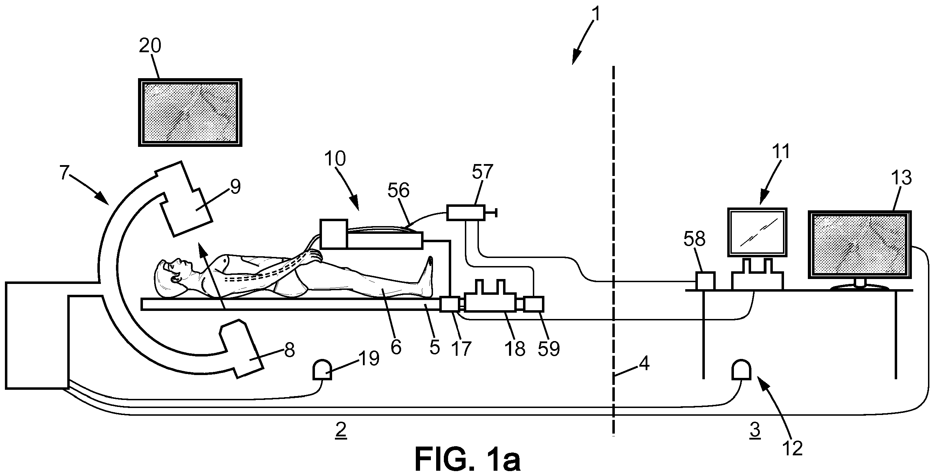

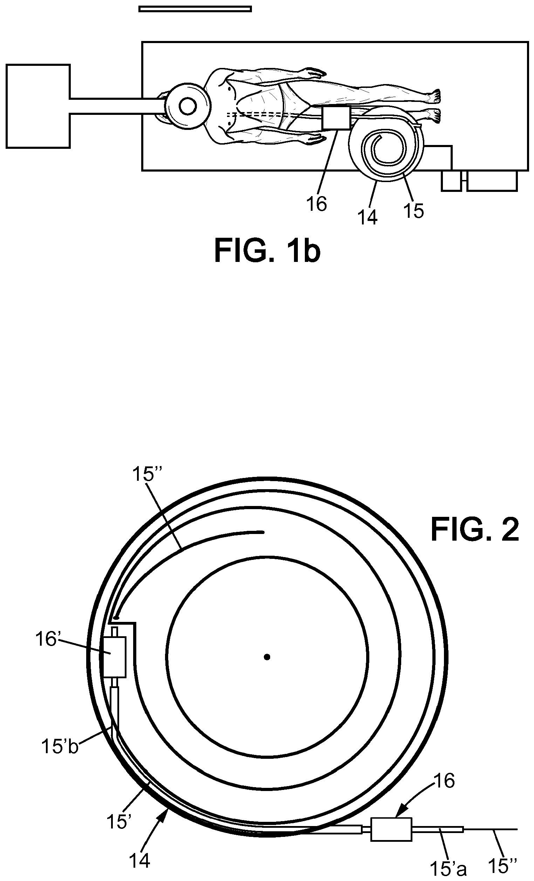

La figure 1a représente schématiquement une installation d'artériographie 1. L'installation d'artériographie 1 est divisée en deux endroits distincts, une salle d'opérations 2 et une salle de commande 3. La salle de commande 3 peut être proche de la salle d'opération 2, séparée de celle-ci par une simple paroi 4, opaque aux rayons X, ou distante. Les matériels de la salle d'opération 2 et de la salle de commande 3 sont reliés entre eux de manière fonctionnelle, par voie filaire, sans fil ou réseau, ....

La salle d'opérations 2 comprend une table d'opérations 5 recevant un patient 6. La salle d'opérations 2 peut également comprendre un imageur médical 7, notamment un imageur par rayons X, comprenant une source 8 et un détecteur 9 disposés de part et d'autre du patient, éventuellement mobiles par rapport au patient.

L'installation d'artériographie 1 comprend un robot 10 disposé dans la salle d'opérations 2.

L'installation d'artériographie 1 comprend un poste de commande 11 disposé dans la salle de commande 3. Le poste de commande 11 est adapté pour commander à distance le robot 10. L'installation d'artériographie 1 peut également comprendre, disposée dans la salle de commande 3, une ou plusieurs commandes distantes 12 de l'imageur 7, communiquant avec l'imageur 7 pour commander celui-ci à distance. L'installation d'artériographie 1 peut également comprendre, disposé dans la salle de commande 3, un écran 13, communiquant avec l'imageur 7, pour visualiser en temps réel dans la salle de commande 3 les images acquises par l'imageur 7.

Le robot 10 peut comprendre un récipient 14 adapté pour contenir un organe médical souple allongé 15 à introduire dans le corps d'un patient. A titre d'organe médical souple allongé 15, il peut par exemple s'agir d'un organe à introduire dans un canal d'un patient, et à déplacer dans ce canal, notamment une artère ou une veine d'un patient, à travers un désilet ménageant une ouverture d'accès dans le patient. L'organe médical souple allongé peut être notamment un cathéter. En variante, l'organe médical souple allongé peut être un guide pour cathéter. Un guide est généralement de diamètre transversal inférieur à celui du cathéter, qui est généralement creux sur une portion proche du patient, voire sur la totalité de sa longueur, de sorte que le guide puisse se déplacer à l'intérieur de celui-ci, notamment à l'intérieur du corps du patient. Le guide peut également comporter une extrémité recourbée, comme il sera décrit plus en détail ci-après.

Le robot 10 peut comprendre un module d'entraînement 16 de l'organe médical souple allongé 15. Le module d'entraînement 16 est commandable à partir du poste de commande 11 pour entraîner l'organe médical souple allongé par rapport au patient selon au moins un degré de liberté, comme ce sera décrit en détails par la suite. Le module d'entraînement peut comprendre un boîtier de communication 17 servant à l'interfaçage avec le poste de commande 11. Au besoin, le robot 10 peut comprendre un boîtier de commande 18 en local, destiné à commander le robot depuis la salle d'opérations 2 si nécessaire.

On notera d'ailleurs que toutes les commandes et les retours disponibles dans la salle de commande 3 peuvent être également disponibles dans la salle d'opérations 2 en vue d'une opération en local, comme par exemple une commande 19 de l'imageur et un écran 20 permettant de visualiser les images acquises par l'imageur 7.

L'organe médical souple allongé 15 creux peut être raccordé à un raccord 56 permettant l'injection d'un produit de contraste facilitant l'imagerie à l'intérieur de l'organe médical souple allongé. L'installation d'artériographie peut comprendre un injecteur 57 de produit de contraste raccordé au raccord 56, commandable par une commande 58 disposée dans la salle de commande 3. Une commande 59 de l'injecteur de produit de contraste peut également être présente en local dans la salle d'opérations 2.

Comme visible sur la figure 2, de manière purement illustrative, on a représenté plus en détail le récipient 14 recevant un cathéter 15'. Le récipient 14 permet de maintenir le cathéter 15' dans un milieu convenant à sa conservation. Le module d'entraînement 16 est adapté pour l'entraînement du cathéter 15'. Dans l'exemple, on prévoit que le récipient 14 reçoit également un guide 15''. Le récipient 14 permet de maintenir le guide 15'' dans un milieu convenant à sa conservation. Le module d'entraînement 16' est adapté pour l'entraînement du guide 15''. Selon les applications, les modules d'entraînement 16, 16', peuvent être identiques ou différents. Ils peuvent être selon l'un des modes de réalisation présentés ci-dessous, si approprié. Dans l'exemple présenté, le guide 15'' peut être introduit dans le cathéter 15' à l'extrémité arrière 15'b de celui-ci, et dépasser de l'extrémité avant 15'a du cathéter comme représenté.

Dans ce qui suit, on utilisera la référence 15 pour désigner alternativement le guide 15'', le cathéter 15', ou de manière générale un organe médical souple allongé à introduire dans le corps d'un patient. Il peut par exemple s'agir d'un cathéter interventionnel. Un tel cathéter interventionnel peut être de diamètre inférieur au cathéter, de manière à être guidé à l'intérieur de celui-ci, coaxialement à l'intérieur du patient, et être creux de manière à être guidé sur le guide à l'intérieur du patient.

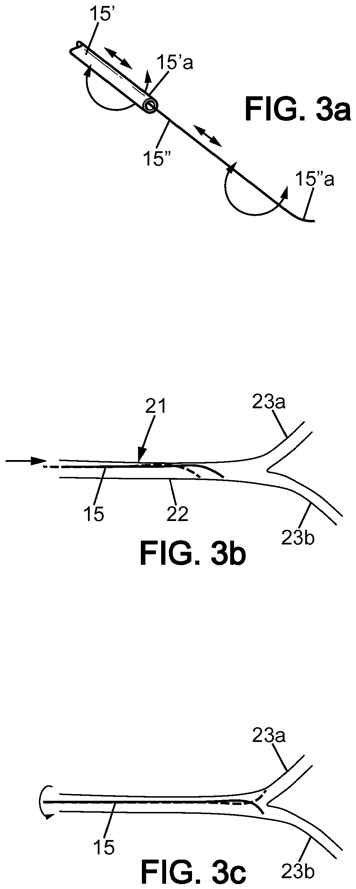

La figure 3a représente les divers degrés de liberté envisageables avec le présent système. On visualise le guide 15" avec son extrémité avant 15"a légèrement courbée par rapport à l'axe longitudinal principal du guide, et débouchant par l'extrémité avant 15'a du cathéter 15'. Le cathéter 15' peut être soumis à deux mouvements distincts :
- Une translation selon son axe longitudinal,
- Une rotation autour de son axe longitudinal.

Ces mouvements peuvent être générés dans un sens ou dans l'autre.

Le cas échéant, le cathéter 15' peut être soumis à un mouvement combiné des deux mouvements simples décrits ci-dessus.

Le cas échéant, le cathéter 15' peut être soumis à deux mouvements combinés des deux mouvements simples décrits ci-dessus, selon des combinaisons différentes.

Ce qui a été décrit ci-dessus concernant le cathéter s'applique également au guide.

Le guide 15'' peut être soumis à deux mouvements distincts :
- Une translation selon son axe longitudinal,
- Une rotation autour de son axe longitudinal.

Ces mouvements peuvent être générés dans un sens ou dans l'autre.

Le cas échéant, le guide 15'' peut être soumis à un mouvement combiné des deux mouvements simples décrits ci-dessus.

Le cas échéant, le guide 15'' peut être soumis à deux mouvements combinés des deux mouvements simples décrits ci-dessus, selon des combinaisons différentes.

Dans certains cas, le cathéter est lui-même pourvu d'une extrémité courbe, soit pour permettre la navigation sur le même principe qu'un guide, soit pour faciliter le positionnement dans une zone anatomique présentant une courbure particulière.

Sur la figure 3b, on a représenté une artère 21 d'un patient comprenant un tronc principal 22 et deux branches 23a, 23b débouchant sur le tronc principal. La figure 3b illustre le déplacement d'un organe médical souple allongé 15 (ici un guide 15'') selon une translation entre une position reculée représentée en pointillés et une position avancée représentée en traits pleins. Sur la figure 3c, dans la même artère, on a représenté une rotation de l'organe médical souple allongé 15 entre une première position, représenté en pointillés, où l'organe médical souple allongé est prêt à être soumis à une translation en direction de la branche 23a, et une deuxième position, représentée en traits pleins, où l'organe médical souple allongé est prêt à être soumis à une translation en direction de la branche 23b.

L'organe médical souple allongé peut être entraîné selon le ou les déplacements décrits ci-dessus par des organes d'entraînement. Les organes d'entraînement peuvent être agencés par paires.

Selon un exemple de réalisation, un organe d'entraînement donné peut être actionné par un actionneur.

On a ainsi présenté un système d'actionnement 55, 55', 55'' d'un doigt d'entraînement 24 selon 3 directions indépendantes de l'espace.

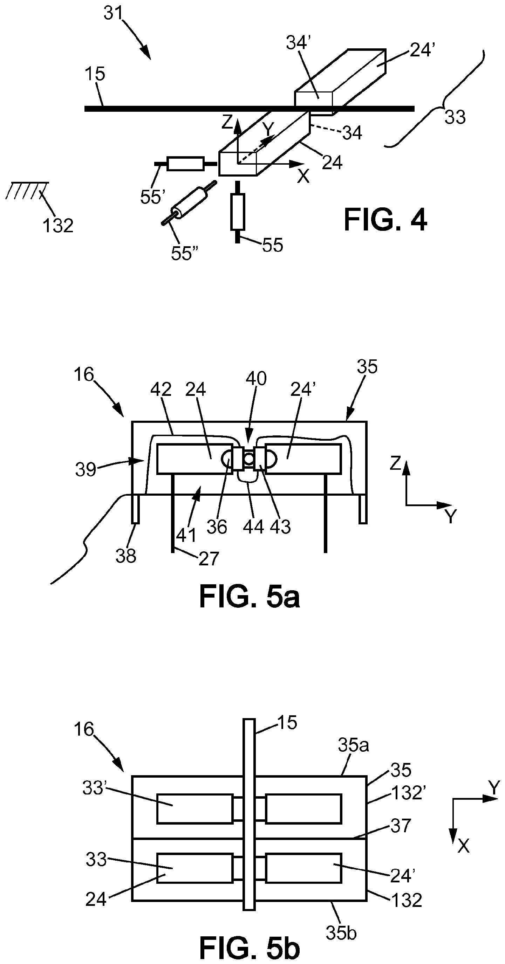

Sur la figure 4, on a représenté un module d'entraînement 31 selon un premier mode de réalisation. Ce module d'entraînement 31 est adapté pour entraîner un organe médical souple allongé 15 s'étendant selon une direction longitudinale X. On notera que la direction longitudinale X au niveau du module d'entraînement 31 n'est pas forcément la même que celle de l'organe médical souple allongé 15 au niveau de son extrémité mais qu'une translation et/ou une rotation de l'organe médical souple allongé 15 selon/autour de la direction longitudinale X au niveau du module d'entraînement 31 entraînera une translation et/ou une rotation de l'organe médical souple allongé 15, respectivement, selon/autour sa direction longitudinale au niveau de son extrémité.

Le module d'entraînement 31 comprend une base 132 et au moins un organe d'entraînement 24 monté mobile par rapport à la base 132. L'organe d'entraînement 24 est par exemple monté mobile par rapport à la base 132 selon trois degrés de liberté.

Dans l'exemple présenté, le module d'entraînement 31 comprend en outre un deuxième organe d'entraînement 24'. L'organe d'entraînement 24, aussi appelé par la suite premier organe d'entraînement, et le deuxième organe d'entraînement 24' forment ensemble une paire d'organes d'entraînement 33. Une paire d'organes d'entraînement 33 comprend deux organes d'entraînement qui coopèrent ensemble pour générer un mouvement de l'organe médical souple allongé 15 par rapport à la base 132. Dans l'exemple présenté, le deuxième organe d'entraînement 24' est monté mobile par rapport à la base 132. Le deuxième organe d'entraînement 24' est par exemple monté mobile par rapport à la base 132 selon trois degrés de liberté.

Le premier organe d'entraînement 24 et le deuxième organe d'entraînement 24' sont appariés pour des mouvements simultanés. Par exemple, les premier et deuxième organes d'entraînement 24, 24' peuvent être commandés individuellement indépendamment l'un de l'autre, mais selon des commandes respectives synchronisées. En variante, on peut prévoir une commande commune qui va être distribuée à l'un et à l'autre des premier et deuxième organes d'entraînement 24, 24' par une liaison mécanique ou électronique entre leurs systèmes de commande.

Chaque organe d'entraînement 24, 24' comporte une surface d'entraînement 34, 34' respectivement. L'organe médical souple allongé 15 est disposé entre les surfaces d'entraînement 34, 34' des organes d'entraînement 24, 24' d'une même paire. Pour fixer les idées, les surfaces d'entraînement 34, 34' sont espacées l'une de l'autre selon la direction Y.

La paire d'organes d'entraînement 24, 24' peut être placée dans une configuration libre, représentée sur la figure 4, dans laquelle la surface d'entraînement 34, 34' des organes d'entraînement 24, 24' de la paire d'organes d'entraînement 33 n'est pas en prise avec l'organe médical souple allongé 15.

La paire d'organes d'entraînement 33 est plaçable dans une configuration d'entraînement dans laquelle les surfaces d'entraînement 34, 34' des organes d'entraînement de la paire d'organes d'entraînement sont en prise avec l'organe médical souple allongé 15 à entraîner. La force appliquée par un organe d'entraînement sur l'organe médical souple allongé dans cette configuration est par exemple de l'ordre de quelques Newtons (5-30 N par exemple). Des moyens de rappel sont par exemple agencés pour ramener la paire d'organes d'entraînement en configuration libre, ce qui permet de fournir une fonction sécuritaire, par exemple en cas de rupture d'alimentation électrique.

Pour placer la paire d'organes d'entraînement 33 alternativement dans les configurations libre et d'entraînement, on peut commander un déplacement relatif l'un vers l'autre des deux organes d'entraînement 24, 24'. Ce déplacement peut par exemple être le déplacement d'un organe d'entraînement 24 par rapport à la base, l'autre restant fixe. En variante, les deux organes d'entraînement 24, 24' peuvent tous deux se déplacer l'un vers l'autre par rapport à la base.

Dans l'exemple, on prévoit un déplacement selon la direction Y.

Dans le mode de réalisation présenté, les deux organes d'entraînement 24, 24' sont mobiles par rapport à la base selon un degré de liberté. Ce degré de liberté diffère de celui permettant le placement alternatif des organes d'entraînement entre les positions libre et d'entraînement. On prévoit notamment que les organes d'entraînement 24, 24' sont mobiles par rapport à la base selon un degré de liberté dans leur configuration d'entraînement. Ainsi, le déplacement des organes d'entraînement selon un degré de liberté dans leur configuration d'entraînement génère un déplacement de l'organe médical souple allongé par rapport à la base 132.

Selon un exemple, on décrit la génération d'un mouvement de translation de l'organe médical souple allongé selon sa direction longitudinale X.

La position de départ correspond à celle de la figure 4 décrite ci-dessus. Dans un premier temps, on passe de la configuration libre à la configuration d'entraînement. Selon l'exemple, ce passage se fait par un mouvement en sens opposé des deux organes d'entraînement selon la direction Y. L'amplitude de ce mouvement peut dépendre de l'organe médical souple allongé 15 à entraîner. Un guide, de diamètre inférieur au cathéter, pouvant nécessiter un mouvement de plus grande amplitude que le cathéter à partir d'une même position de départ.

En configuration d'entraînement, on génère un déplacement simultané dans le même sens des organes d'entraînement selon la direction longitudinale X selon un premier sens, ce qui génère un mouvement identique de l'organe médical souple allongé 15.

On passe de la configuration d'entraînement à la configuration libre. Selon l'exemple, ce passage se fait par un mouvement en sens opposé des deux organes d'entraînement selon la direction Y, dans le sens opposé au sens pour faire passer les organes d'entraînement de la configuration libre à la configuration d'entraînement.

En configuration libre, on génère un déplacement simultané (ou non) dans le même sens des organes d'entraînement selon la direction longitudinale X selon un deuxième sens opposé au premier sens, ce qui ne génère pas de mouvement de l'organe médical souple allongé 15. On est alors revenu à la configuration de départ.

On peut répéter les étapes ci-dessus de manière commandée cyclique pour générer une translation de l'organe médical souple allongé selon une course longue (par exemple de l'ordre de plusieurs mètres) selon la direction longitudinale X dans le premier sens.

Le déplacement de l'organe médical souple allongé selon une course longue selon la direction longitudinale X dans le deuxième sens peut se faire par une suite d'opérations opposées de celle qui vient d'être décrite.

La fréquence du cycle peut être réglable et commandable. En particulier, on peut prévoir une fréquence faible pour l'introduction de l'organe médical souple allongé dans le patient, voire plusieurs niveaux de fréquence faible, pour permettre en particulier une navigation lente dans les environnements difficiles. On peut prévoir une fréquence rapide, par exemple pour un retrait, voire un retrait d'urgence. Les amplitudes de déplacement pour chaque cycle peuvent également être réglables.

Pour la translation, des vitesses comprises entre 0,1 et 200 millimètres par secondes sont envisageables.

Selon un exemple, on décrit la génération d'un mouvement de rotation de l'organe médical souple allongé autour de sa direction longitudinale X.

La position de départ correspond à celle de la figure 4 décrite ci-dessus. Dans un premier temps, on passe de la configuration libre à la configuration d'entraînement. Selon l'exemple, ce passage se fait par un mouvement en sens opposé des deux organes d'entraînement selon la direction Y. Ce passage est le même que déjà décrit ci-dessus.

En configuration d'entraînement, on génère un déplacement simultané en sens opposé des organes d'entraînement selon une direction Z transversale à la direction longitudinale X, différente de la direction Y, ce qui génère un mouvement de rotation de l'organe médical souple allongé 15 autour de la direction longitudinale X. En particulier, l'organe médical souple allongé roule, de préférence sans glissement, sur les surfaces d'entraînement 34, 34' des organes d'entraînement 24, 24'. En variante, on pourrait déplacer un seul des deux organes d'entraînement, l'autre restant fixe.

On passe de la configuration d'entraînement à la configuration libre. Selon l'exemple, ce passage se fait par un mouvement en sens opposé des deux organes d'entraînement selon la direction Y, dans le sens opposé au sens pour faire passer les organes d'entraînement de la configuration libre à la configuration d'entraînement.

En configuration libre, on génère un déplacement simultané (ou non) des organes d'entraînement selon la direction Z, opposé au déplacement décrit ci-dessus, ce qui ne génère pas de mouvement de l'organe médical souple allongé 15. On est alors revenu à la configuration de départ.

On peut répéter les étapes ci-dessus de manière commandée cyclique pour générer une rotation de l'organe médical souple allongé selon une course longue (par exemple de plusieurs fois 360°) autour de la direction longitudinale X dans un premier sens de rotation.

Le déplacement de l'organe médical souple allongé selon une course longue autour de la direction longitudinale X dans le deuxième sens de rotation opposé au premier peut se faire par une suite d'opérations opposées de celle qui vient d'être décrite.

Dans les deux exemples de réalisation ci-dessus, on a décrit un déplacement séquencé au cours duquel on attend d'avoir terminé le déplacement d'un organe d'entraînement selon une direction pour entamer un autre déplacement.

Toutefois, étant donné que les actionnements des organes d'entraînement selon divers degrés de liberté peuvent être rendus indépendants en utilisant de manière indépendante les trois systèmes d'actionnement 55, 55', 55'' décrits plus haut, on pourrait mettre en oeuvre de manière simultanée le déplacement d'un organe d'entraînement selon deux degrés de liberté. Par exemple, le déplacement des organes d'entraînement entre la fin de la course d'entraînement et le retour en position initiale pourrait inclure une phase intermédiaire entre une première phase de pur écartement et une deuxième phase de pur retour à la position initiale, où ces deux mouvements sont combinés. Une phase intermédiaire similaire est également envisageable entre la position écartée de la fin de la course d'entraînement et la position en prise au niveau du début de la course d'entraînement entre la phase de pur retour à la position initiale et une phase de pur rapprochement. En tirant le trait, on pourrait n'avoir plus de phases de pur retour à la position initiale, pur écartement et pur rapprochement, dans la mesure où on ne risque pas de générer de mouvements parasites de l'organe médical souple allongé.

Par ailleurs, alors qu'on a présenté de manière indépendante un mouvement de translation pure de l'organe médical souple allongé et un mouvement de rotation pure, ces deux mouvements pourraient alternativement être combinés. Il suffirait, en configuration en prise, de combiner les mouvements adaptés des organes d'entraînement pour générer simultanément translation et rotation.

L'exemple précédent comprend une unique paire d'organes d'entraînement.

En variante, on pourrait prévoir plusieurs paires d'organes d'entraînement. Par exemple, à titre descriptif, on pourrait prévoir deux paires d'organes d'entraînement. Les organes d'entraînement 24", 24‴ de la deuxième paire 33' peuvent être similaires à ceux de la première paire, et en particulier comporter des surfaces d'entraînement 34", 34''', et être actionnés depuis le poste de commande 11 distant, voire le boîtier de commande 18 local selon des mises en oeuvre similaires à celles de la première paire. La première paire 33 et la deuxième paire 33' d'organes d'entraînement peuvent être décalées l'une par rapport à l'autre selon l'axe longitudinal X de l'organe médial souple allongé. Selon un premier exemple, les deux paires 33, 33' peuvent être prévues coplanaires dans leur configuration libre. C'est-à-dire qu'elles peuvent être prévues vis-à-vis d'une base 132 commune aux deux paires. En variante, les bases 132, 132' de chaque paire pourraient être indépendantes, voire non coplanaires.

Les actionnements des deux paires peuvent être synchronisés. Par exemple, les actionnements des deux paires peuvent générer des mouvements identiques simultanés des deux paires.

En variante, les deux paires peuvent être actionnées de manière synchronisées pour générer des mouvements décalés en phase. C'est-à-dire qu'une première paire 33 peut être en configuration d'entraînement pendant qu'une autre paire est en configuration libre, et vice-versa. Par exemple, il y a toujours au moins une paire en configuration d'entraînement. A chaque moment donné, il peut s'agir de la première, de la deuxième, voire des deux en même temps. Une telle configuration permet d'améliorer le maintien de l'organe médical souple allongé. Notamment quand l'organe médical souple allongé est déplacé en frottant contre une zone anatomique du patient, il faut pouvoir assurer un maintien suffisant de celui-ci pour vaincre la résistance locale au déplacement. Ceci est rendu d'autant plus difficile quand l'organe médical souple allongé est glissant, par exemple du fait de son maintien dans une solution.

Dans les modes de réalisation décrits ci-dessus, les organes d'entraînement sont disposés de manière symétrique par rapport à un plan général médian de l'organe médical souple allongé.

Toutefois, en variante, les organes d'entraînement peuvent être montés mobiles par rapport à la base 132 pour décaler latéralement l'organe médical souple allongé localement par rapport à son axe longitudinal neutre X'. L'axe longitudinal neutre X' est défini par l'axe longitudinal naturellement occupé par l'organe médical souple allongé sans aucune sollicitation par des organes d'entraînement 24. Un tel décalage latéral est possible en générant un mouvement simultané des organes d'entraînement 24, 24' en configuration en prise dans le même sens selon une direction transversale (axe Y ou Z, ou une combinaison selon ces deux axes) par rapport à la configuration en prise au niveau de l'axe longitudinal neutre.

Le cas échéant, si on utilise plusieurs paires d'organes d'entraînement, celles-ci peuvent être disposées, en configuration en prise, selon des décalages latéraux différents par rapport à l'axe longitudinal neutre. On peut alors mettre en oeuvre un actionnement de type « manivelle ».

Les figures 5a et 5b présentent un module robotisé d'entraînement 16 comprenant un boîtier 35 logeant les organes d'entraînement 24, 24', 24", 24'''. Le boîtier 35 présente des ouvertures autorisant le passage de tiges d'actionnement 27 (seule la tige d'actionnement selon Z est représentée sur la figure 5a), dont l'autre extrémité est solidaire d'un actionneur (non représenté). En variante, on notera que la tige, représentée sur la figure 5b avec la référence 27, n'est pas nécessairement une tige d'actionnement, mais peut simplement être une tige de transfert de mouvement, solidaire à une extrémité de l'organe d'entraînement 24. Dans ce cas, c'est l'autre extrémité de cette tige de transfert de mouvement qui est soumis à l'actionnement par les systèmes d'actionnement 55, 55', 55". Le boîtier 35 comporte également une face amont 35a et une face aval 35b comprenant chacune une lumière 36 permettant le passage de l'organe médical souple allongé. La lumière 36 peut être réalisée sous la forme d'une fente pour permettre le retrait d'urgence de l'organe médical souple allongé.

Si, selon l'exemple présenté, on utilise plusieurs paires d'organes d'entraînement décalées l'une par rapport à l'autre selon l'axe longitudinal X, deux paires d'organes d'entraînement 33, 33' successives peuvent être séparées par une paroi intermédiaire 37 du boîtier. La paroi intermédiaire 37 peut également comporter une lumière pour le passage de l'organe médical souple allongé, cette lumière étant délimitée par un bord pouvant former une zone de support de l'organe médical souple allongé.

Le boîtier 35 comprend un système de fixation 38 pour la fixation du boîtier 35 à un support, par exemple solidaire du récipient 14. Tout type de fixation est envisageable, amovible ou non, par exemple par clipsage ou par verrouillage électromécanique.

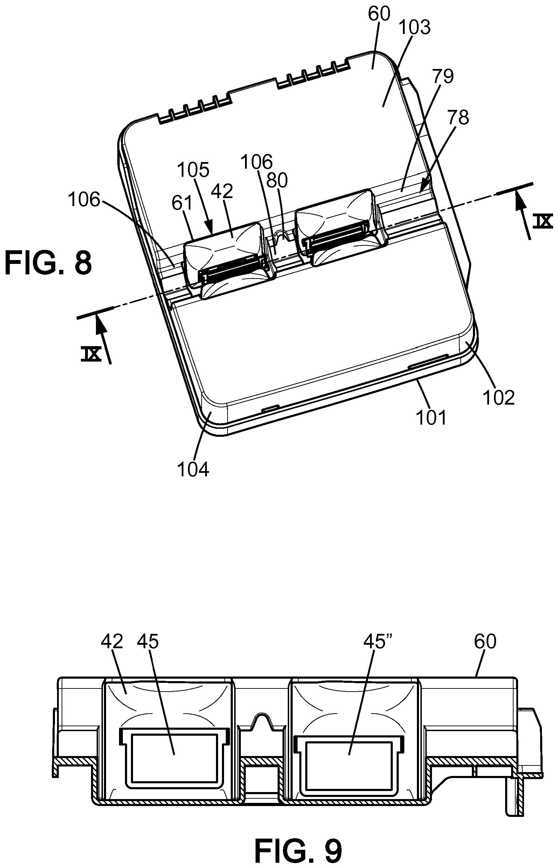

Le boîtier 35 comprend également une barrière stérile 39 qui sera décrite plus en détail ci-après. Notamment, le boîtier 35 comprend une portion de base à demeure 101, et une cassette 102 consommable à usage unique. La portion de base 102 porte les organes d'entraînement 24-24‴. La cassette 102 peut être assemblée à/désassemblée de la portion de base à demeure 101 par tout mécanisme appropriée. Comme représenté sur la figure 8, dans un exemple, la cassette 102 coopère par emboîtement (complémentarité de forme) avec la portion de base à demeure 101. La cassette 102 comporte la barrière stérile 39. La barrière stérile 39 peut être solidaire du boîtier 35. La barrière stérile 39 sépare l'espace en deux sous-espaces, un sous-espace stérile 40 comprenant notamment l'organe médical souple allongé 15, et un sous-espace pas nécessairement stérile 41. Dans l'exemple présenté, les organes d'entraînement 24, 24' sont disposés dans le sous-espace pas nécessairement stérile 41. C'est-à-dire que les organes d'entraînement 24, 24' coopèrent avec l'organe médical souple allongé à travers la barrière stérile 39.

Par exemple, la barrière stérile 39 comprend un film 42 continu comprenant une portion d'attachement 43 pour chaque organe d'entraînement 24, 24', et des portions souples 44 entre deux portions d'attachement voisines. Les portions souples 44 sont par exemple translucides. Chaque organe d'entraînement 24, 24' est attaché à une portion d'attachement 43 du film 42 continu, les surfaces d'entraînement 34, 34' étant en prise avec l'organe médical souple allongé 15 par l'intermédiaire du film 42 continu.

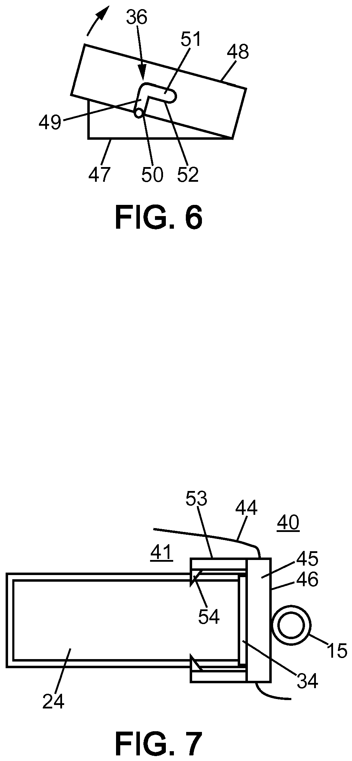

Comme cela est visible sur la figure 7, on prévoit par exemple que les portions souples 44 sont solidaires d'un pion d'accrochage 45. Les portions souples 44 sont par exemple surmoulées sur les pions d'accrochage 45. Le pion d'accrochage 45 est assemblable par tout moyen approprié à l'organe d'entraînement 24, par l'intermédiaire d'une portion d'assemblage 54, tel que par exemple par clipsage dans le sous-espace pas nécessairement stérile 41 (auquel cas la portion d'assemblage est une portion de clipsage). La surface d'entraînement 46 du pion d'accrochage 45, en contact avec l'organe médical souple allongé 15, peut être spécialement adaptée à ce contact. Par exemple, elle est formée, striée, molletonnée et/ou présente un revêtement adapté à ce contact.

Les portions souples 44 sont suffisamment longues et souples pour permettre les mouvements relatifs décrits ci-dessus pour les organes d'entraînements 24, 24' sans empêchement ou sans détérioration de la barrière stérile 39.

Les pions d'accrochage 45 peuvent comprendre des zones frangibles 53 tels que des bras reliant la surface d'entraînement 46 à la portion de clipsage. Après une intervention, lorsqu'on retire la barrière stérile, on brise les zones frangibles 53. Ce mécanisme offre une sécurité pour empêcher, le cas échéant, de réinstaller une barrière stérile usagée pour une intervention ultérieure.

Selon une variante, comme représenté sur la figure 8, la barrière stérile 39 comporte un carter 60 rigide doté de fenêtres 61. On prévoit par exemple une fenêtre 61 par organe d'entraînement 24. Les fenêtres 61 sont notamment ménagées dans des parois latérales 79 d'un canal 78 du carter, s'étendant sensiblement selon la direction X. Ainsi, le carter 60 comprend une paroi supérieure 103, par exemple plane, dans laquelle est ménagée le canal 78, et une jupe périphérique 104 servant à emboîter le carter 60 sur un composant à demeure. Le canal 68 comporte une zone de fenêtres 105 dans laquelle sont disposées deux fenêtres 61 en vis-à-vis, et une zone sans fenêtres 106 adjacente à la zone à fenêtres 105. Dans le cas présent où on utilise deux paires d'organes d'entraînement, le canal comporte deux zones à fenêtres 105, et une zone sans fenêtre 106 entre ces deux zones à fenêtres 105. Le canal présente en outre une zone sans fenêtres 106 arrière et une zone sans fenêtre avant (les termes « arrière » et « avant » étant utilisés par référence au patient). Le canal 68 peut également comporter un relief localisé 80 formant détrompeur. Les dimensions de la fenêtre 61 selon les directions X et Z sont largement supérieures à la course des organes d'entraînement 24 selon ces directions, respectivement. On décrira ensuite une fenêtre 61, sachant que la même description s'applique aux autres fenêtres. Un film 42 souple est solidarisé au pourtour de chaque fenêtre, et ferme de manière étanche chaque fenêtre. Un pion d'accrochage 45 s'étend dans chaque fenêtre 61. Le pion d'accrochage 45 est suspendu au carter 60 par l'intermédiaire du film souple 42. Le film 42 souple est solidarisé au pourtour du pion d'accrochage 45 de manière étanche. La flexibilité du film souple 42 autorise un déplacement du pion d'accrochage 45 selon une certaine plage, dans chacune des trois directions de l'espace, en conservant l'étanchéité de l'assemblage du pion d'accrochage 45 au carter 60. Ainsi, le pion d'accrochage 45 est relié au carter 60 de manière étanche par des portions souples 44.

La figure 9 représente ainsi les pions d'accrochage 45 et 45'' dans des positions différentes selon les axes X et Z. Les pions d'accrochage 45 et 45'' sont également dans des positions différentes selon l'axe Y, ce qui n'est pas visible sur cette vue en coupe. La flexibilité du film souple 42 autorise ces déplacements.

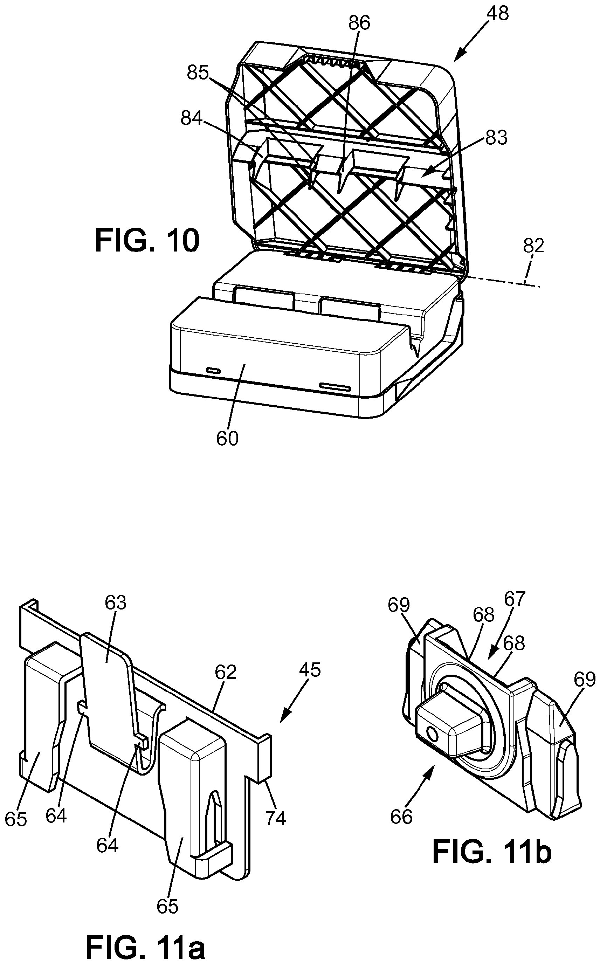

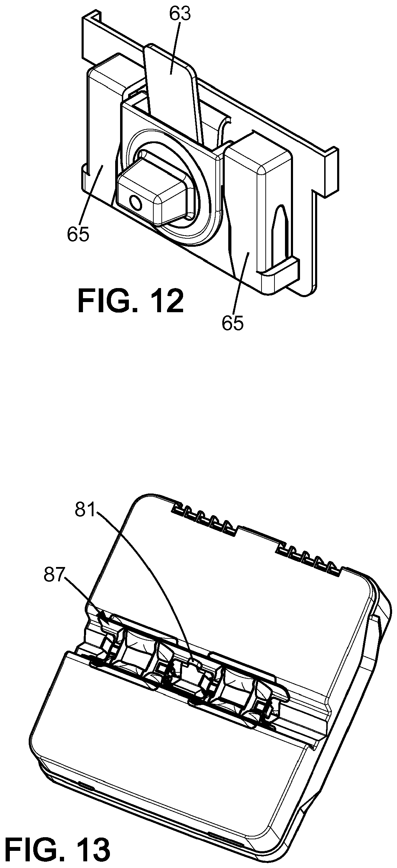

Les figures 11a, 11b et 12 présentent un exemple d'assemblage du pion d'accrochage 45 à l'organe d'entraînement 24. La figure 11a représente le pion d'accrochage 45 (le film souple 42 n'est pas représenté). Le pion d'accrochage 45 comprend une paroi plane 62 à l'arrière de laquelle est monté un système d'assemblage amovible à l'organe d'entraînement. A l'avant de la paroi plane 62, on prévoit la surface d'entraînement 46 en contact avec l'organe médical souple allongé (« avant » et « arrière » étant utilisés par référence à l'organe médical souple allongé). Le système d'assemblage amovible comprend une languette élastique 63 portant deux ergots de verrouillage 64. Le système d'assemblage amovible comporte également un système de guidage. Le système de guidage comprend deux parties symétriques l'une de l'autre et disposées de part et d'autre de la languette élastique 63. Chaque partie comprend une paroi de guidage 65 parallèle et en regard de la paroi plane 62. Le système d'assemblage amovible est ouvert par le bas, afin de permettre son assemblage depuis le haut sur un embout 66 de l'organe d'entraînement 24.

L'embout 66 est représenté sur la figure 11b. Il est solidaire en déplacement de l'extrémité de l'organe d'entraînement 24. Il comporte une fenêtre avant 67 capable de recevoir la languette élastique 63. Deux butées 68 sont ménagées dans la fenêtre avant. Deux renforts latéraux 69 sont disposés de part et d'autre de la fenêtre avant 67. Les renforts latéraux 69 sont de formes complémentaires aux parties du système de guidage.

Le pion d'accrochage 45 s'assemble à l'embout 66 par coulissement depuis le haut. Les renforts 69 s'insèrent dans les parties respectives du système de guidage, en y étant serrés, et la languette élastique 63 dans la fenêtre avant 67, en étant déformée élastiquement. Une fois la position d'assemblage atteinte, la languette élastique 63 est partiellement relâchée, autorisant les ergots 64 à venir coopérer avec les butées 68. Cette coopération empêche un déplacement vers le haut du pion d'accrochage par rapport à l'embout 66. Le pion d'accrochage 45 et l'embout 66 sont solidarisés l'un à l'autre.

Dans le mode de réalisation ci-dessus, le film souple 42 étant flexible, il peut être nécessaire d'assembler un à un chaque pion d'accrochage 45 à l'embout 66 correspondant, ce qui peut être difficile, du fait de la petite taille des composants, de l'environnement stérile, du faible espace disponible, étant donné qu'il est nécessaire de ne pas abimer le film souple 42.

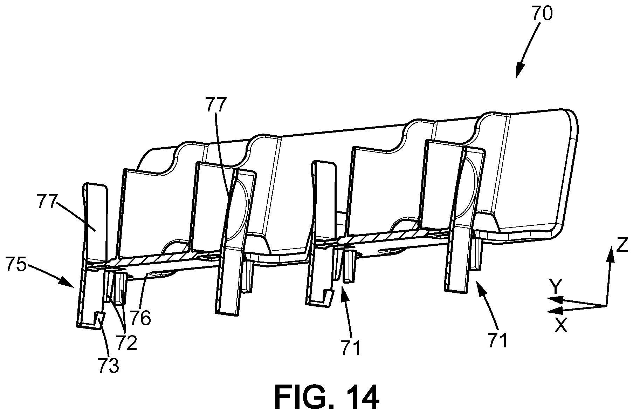

En variante optionnelle, on prévoit un véhicule 87 pour le maintien temporaire des pions 45. Selon ce mode de réalisation, le véhicule met en oeuvre une réglette 70 amovible. La réglette 70 est représentée sur la figure 14, dans une vue en coupe transversale médiane. La réglette 70 présente une forme générale complémentaire de la forme du carter 60, notamment du canal central 78 du carter 60. Elle peut s'emboîter dans celui-ci dans une unique position donnée. On prévoit ainsi un relief 81 (figure 13) localisé complémentaire du relief 80 du canal 68. Un pion 45 est maintenu par deux régions de maintien 71 de la réglette, les deux régions de maintien 71 étant espacées l'une de l'autre selon la direction X. Le pion 45 est maintenu selon la direction Y par des ergots 72 à disposer de part et d'autre de la paroi plane 62. Par exemple, on prévoit une paire d'ergots 72 par région de maintien. Une butée 73 participe au maintien du pion 45 selon la direction Z. On prévoit par exemple une telle butée 73 dans chaque région de maintien 71. Chaque butée 73 coopère avec une butée complémentaire 74 ménagée sur le pion 45.

La butée 73 est montée sur une languette flexible 75. Ainsi, la réglette 70 comprend un corps de base 76 sur lequel sont montées, flexibles, deux languettes flexibles 75, susceptibles d'être soumises à une certaine flexion. Les ergots 72 sont disposés sous le corps de base 75. Les languettes flexibles 75 comprennent une portion d'actionnement 77 accessibles à un utilisateur, pour générer une flexion de la languette flexible 75.

Le pion 45 est ainsi maintenu en position entre quatre ergots 72, et en étant supporté en ses deux extrémités latérales par deux butées 73 coopérant avec les butées 74. L'extension de l'ergot 72 du côté de la face arrière de la paroi plane 62 est limitée, de manière à ne pas gêner l'adhésion du film souple au pion 45.

La réglette 70 est symétrique par rapport au plan X-Z. Une languette flexible 75 porte ainsi une autre butée coopérant avec la butée complémentaire du pion 45 en regard.

Le système qui vient d'être décrit est dupliqué pour une autre paire de pions 45 par translation selon l'axe X, les deux systèmes étant réalisés d'un seul tenant par prolongation du corps de base 76.

La réglette 70 définit ainsi une position très précise pour chacun des pions 45 par rapport au corps de base et, par conséquent, par rapport au carter 60 s'il existe une unique position de montage de la réglette 70 sur le carter 60.

Dans l'exemple présenté, au repos, chacun des embouts 66 est disposé dans une position neutre. Cette position correspond à une position dans laquelle l'embout 66 est assemblé au pion 45 dans la position qu'a le pion 45 maintenu par la réglette 70 dans une position définie par rapport au carter 60.

Dans la configuration de livraison, la réglette 70 est donc assemblée au carter 60 dans la position unique, et supporte les quatre pions 45 dans une position définie.

Les actionneurs sont au repos, de sorte que les embouts 66 sont dans une position définie.

Le carter 60 est amené en position par rapport à la portion de base à demeure 101, et les embouts 66 et les pions 45 s'assemblent les uns aux autres comme expliqué ci-dessus.

Cet assemblage est possible car la réglette 70 définit précisément la position du pion 45 dans l'espace, et en particulier dans une position où il est au bon endroit pour être assemblé à l'embout 66 respectif.

Puis, par actionnement sur les portions d'actionnement 77, la réglette 70 est désassemblée des pions 45, et peut être retiré.

Les pions 45 sont alors montés sur les embouts 66, en étant retenus de manière flottante par les portions souples 44, par une seule opération.

En fin d'opération, pour désassembler le pion 45 de la portion de base à demeure 101, on attrape la languette élastique 63 à travers le film souple (le cas échéant translucide pour faciliter cette opération), et on tire sur la languette 63 afin de désengager les ergots de verrouillage 64 des butées 68. On peut ensuite désolidariser la barrière stérile de la portion de base à demeure 101.

Dans des variantes où on utilise un nombre différent de pions 45, on peut utiliser un mécanisme similaire.

Le boîtier 35 peut comporter un réceptacle 47 et un couvercle 48 mobile par rapport au réceptacle 47 entre une configuration fermée où un accès par un opérateur à l'organe médical souple allongé 15 à l'intérieur du boîtier est interdit, et une configuration ouverte où un accès par un opérateur à l'organe médical souple allongé 15 à l'intérieur du boîtier est possible. La figure 6 représente une position intermédiaire possible entre ces configurations. La lumière 36 peut être ouverte pour permettre ce placement dans deux configurations distinctes. Par exemple, la lumière 36 comporte une forme en L, une première branche 49 comprenant une ouverture 50 permettant de désengager l'organe médical souple allongé 15 du couvercle 48, et une deuxième branche 51 comprenant une surface de support 52 pour l'organe médical souple allongé 15 en configuration fermée.

La figure 10 présente de manière plus précise le couvercle 48 selon un mode de réalisation. Dans cet exemple, le couvercle 48 et la barrière stérile sont solidaires et forment un même consommable unique, la cassette 102. Dans cet exemple, le couvercle 48 est monté pivotant par rapport au carter 60 autour d'un axe de pivotement 82.

La face intérieure du couvercle 48 comporte une portion de guidage 83 de forme partiellement complémentaire de celle du canal 78. En particulier, dans la zone sans fenêtres 106 du canal 78, la portion de guidage 83 tend à réduire au maximum l'espace disponible pour l'organe médical souple allongé. La portion de guidage 83 et le canal 78, quand le couvercle est en configuration fermée, définissent ainsi entre eux un espace, au niveau de la zone sans fenêtres 106, dont la section est similaire à celle de l'organe médical souple allongé. De part et d'autre des zones à fenêtres 105, aux extrémités de celles-ci, la portion de guidage comprend des fourches 84. Les fourches 84 comprennent deux branches 85 inclinées se rejoignant en un sommet 86 en communication avec l'espace sus-mentionné. Les branches 85 sont inclinées de manière à guider l'organe médical souple allongé, dont le placement est plus libre au niveau des fenêtres, vers cet espace.

Dans l'exemple ci-dessus, le couvercle est monté pivotant par rapport au carter autour d'un axe de pivot constant. En variante, on pourrait prévoir un montage du couvercle sur le carter selon une autre cinématique, au moyen d'un mécanisme d'articulation adapté. On peut notamment prévoir une course finale selon un mouvement de translation, notamment selon la direction Z, lors de la fermeture du couvercle. Un tel mouvement est considéré comme plus sur pour éviter de coincer l'organe médical souple allongé entre le carter et le couvercle lors de la fermeture de celui-ci. Notamment, on a recours à un mouvement de fermeture en deux étapes, une course d'approche jusque dans une position intermédiaire, puis une course finale comme décrit ci-dessus. La course d'approche peut être selon un degré de liberté différent de la course finale, par exemple un pivotement selon l'axe X, ou une translation selon un autre axe que l'axe Z. En termes de mécanisme, on peut par exemple prévoir un système de glissières guidant le déplacement du couvercle par rapport au carter selon la trajectoire souhaitée.

Le boîtier 35, le cas échéant comprenant les organes d'entraînement, peut être réalisé comme un consommable à usage unique. En variante, comme expliqué ci-dessus, on réalise un système à usage unique, comprenant la barrière stérile, c'est-à-dire le carter, les pions d'accrochage 45 montés solidaires du carter et flottants par l'intermédiaire des portions souples 44 de film, et éventuellement le couvercle comme un consommable à usage unique qui s'assemble de manière stérile étanche à la partie à demeure du robot.

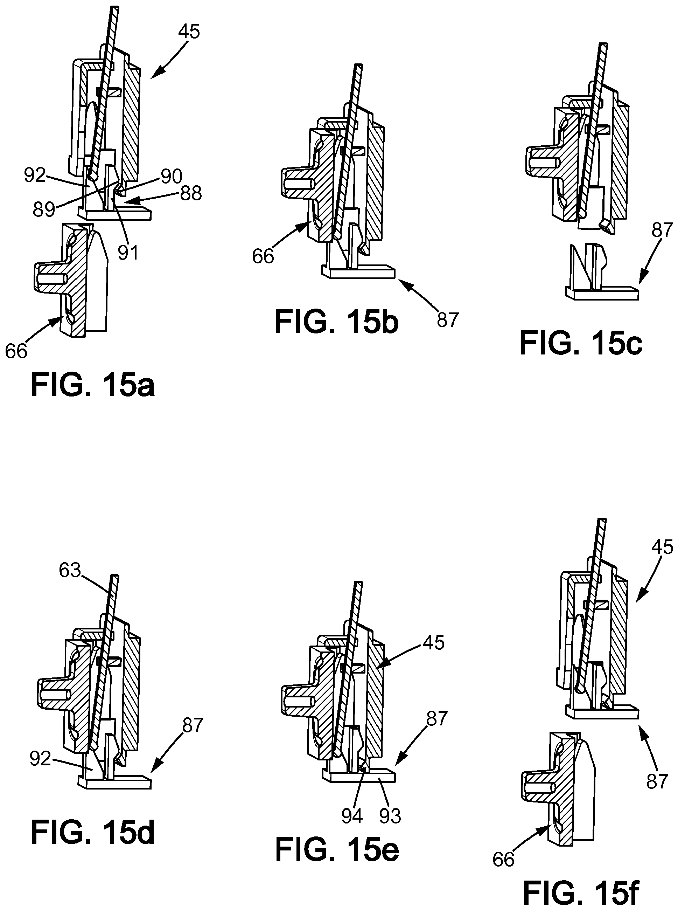

En variante, comme représenté sur les figures 15a-15f, on n'utilise pas nécessairement une réglette 70 amovible à titre de véhicule de montage des pions 45. Dans cet exemple, le véhicule porte-pions 87 reste à demeure sur le carter 60, et est également utilisé pour désassembler les pions 45 des embouts 66 après utilisation. Le véhicule porte-pions 87 comporte un mécanisme de fixation amovible 88 au pion 45. Le mécanisme de fixation amovible 88 comporte une butée 89 qui coopère avec une butée complémentaire 90 du pion 45 pour maintenir ensemble le pion 45 et le véhicule porte-pions 87. Le mécanisme de fixation amovible 88 comprend également une languette flexible 91, portant l'une des butées 89, 90, autorisant une désolidarisation des deux butées, et par conséquent des deux composants. La languette flexible 91 fait par exemple partie du mécanisme de fixation amovible 88.

Le véhicule porte-pions 87 comprend également une portion 92 de désengagement du pion 45 de l'embout 66. On peut par exemple prévoir, à titre de portion 92 de désengagement, un coin pouvant s'insérer dans le mécanisme de verrouillage du pion 45 et de l'embout 66.

La figure 15a représente ainsi une position initiale de l'assemblage du carter à la partie à demeure du robot, dans laquelle le pion 45 est assemblé au véhicule porte-pions 87).

Au cours du mouvement d'assemblage, comme représenté sur la figure 15b, le pion 45 se solidarise sur l'embout 66 par clipsage, comme décrit précédemment. Comme représenté sur la figure 15c, la poursuite du mouvement vers le bas du véhicule porte-pions 87 désolidarise le pion 45, maintenu sur l'embout, du véhicule porte-pions 87, par déformation élastique de la languette flexible 91. Le robot peut alors être utilisé.

Lorsqu'on souhaite retirer le pion 45, comme représenté sur la figure 15d, on applique un mouvement opposé au véhicule porte-pions 87. La portion 92 de désengagement s'insère entre la languette 63 flexible du pion 45 et la portion complémentaire de l'embout 66, et défléchit celle-ci, de manière à désolidariser l'embout 66 du pion 45. La butée 89 peut passer au-delà de la butée 90 par fléchissement de la languette flexible 91. La poursuite du déplacement du véhicule porte-pions 87 entraîne le pion 45 dans une configuration distante de l'embout 66 (figures 15e puis 15f) par appui d'un support 93 du véhicule porte-pions 87 sur une portion 94 du pion 45 en regard.

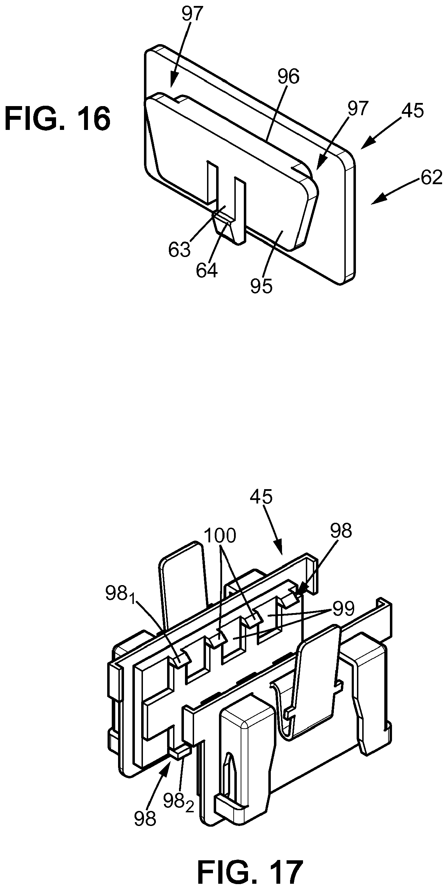

La figure 16 présente une autre variante de pion 45 destiné à coopérer avec un embout 66 complémentaire. Une plaque arrière 95 s'étend parallèlement à la plaque avant 62, une entretoise 96 intermédiaire étant disposée entre la plaque avant 62 et la plaque arrière 95. L'entretoise 96 est moins longue que les plaques avant 62 et arrière 95, de manière à définir deux glissières de guidage 97 de part et d'autre, qui guident un déplacement du pion 45 sur l'embout 66. Pour l'assemblage, une languette de clipsage 63, déformable élastiquement, s'étend vers le bas depuis la plaque arrière 95, et porte un ergot 64.

On peut envisager d'autres technologies de fixation amovible du pion 45 sur l'embout 66 qu'un clipsage, comme par exemple un verrou quart de tour, un aimant ou un électro-aimant.

Selon une réalisation, comme représenté sur la figure 17, on prévoit un système barrière 98 au niveau des pions 45. Le système barrière 98 a pour fonction de limiter un déplacement de l'organe médical souple allongé, au niveau d'une paire de pions 45, selon la direction Z. En effet, un bouclage de l'organe médical souple allongé pourrait conduire à une perte de contact d'avec la paire de pions 45. Le système barrière 98 comprend, pour un pion 45, une barrière supérieure 98₁ et une barrière inférieure 98₂ parallèles. Le cas échéant, chaque barrière 98₁, 98₂ d'un pion 45 est réalisée sous la forme d'une alternance de creux 99 et de saillies 100. Ces barrières et celles du pion opposé sont réalisées en inversion de phase, de sorte qu'un creux de l'une corresponde à une saillie d'une autre. Ainsi, lorsque les deux pions 45 de la même paire sont proches l'un de l'autre, les barrières sont quasiment continues. Cette réalisation permet également de réaliser une fonction barrière avec deux pièces identiques placées en regard l'une de l'autre, ce qui facilite la conception, le nombre de moules, et limite le risque d'erreurs de montage.

La configuration décrite ci-dessus facilite le retrait d'urgence de l'organe médical souple allongé 15 du module robotisé, car celui-ci peut, en toute étape du déplacement, être facilement retiré du module en vue d'une manipulation à la main.

La mise en oeuvre d'une barrière stérile entre l'organe médical souple allongé et les organes d'entraînement est particulièrement bien adaptée au mode de réalisation ci-dessus qui prévoit des mouvements de faible amplitude des organes d'entraînement, ce qui permet de leur attacher la barrière stérile et d'absorber les mouvements par déformation des portions souples. Toutefois, en variante non-revendiqué, on pourrait prévoir d'autres modes de réalisation où la barrière stérile est disposée directement entre l'organe médical souple allongé et les organes d'entraînement, ce qui permet de minimiser les opérations de stérilisation et/ou le nombre de produits consommables à changer entre deux opérations. En variante non-revendiqué, la barrière stérile pourrait donc n'être pas attachée aux organes d'entraînement, mais seulement maintenue de manière adaptée, et les organes d'entraînement pourraient être soumis à des mouvements de grande amplitude.

## Revendications

1. Module robotisé d'entraînement d'organe médical souple allongé comprenant :
- une base (132),
- une paire (33) d'organes d'entraînement (24, 24') présentant chacun une surface d'entraînement (34, 34'), la paire (33) d'organes d'entraînement (24, 24') étant plaçable dans une configuration d'entraînement dans laquelle les surfaces d'entraînement (34, 34') des organes d'entraînement (24, 24') de la paire (33) d'organes d'entraînement (24, 24') sont en prise avec l'organe médical souple allongé à entraîner et disposés de part et d'autre de celui-ci,
la paire (33) d'organes d'entraînement (24, 24') étant montée mobile par rapport à la base (132) selon un degré de liberté entre une première et une deuxième positions,
- un organe de commande (18, 11) adapté pour commander un déplacement par rapport à la base (132) des organes d'entraînement (24, 24') de la paire (33) d'organes d'entraînement (24, 24') en configuration d'entraînement de la première à la deuxième position, entraînant ainsi l'organe médical souple allongé par rapport à la base (132),
- une barrière stérile (39) consommable à usage unique comprenant un pion d'accrochage (45) pour chaque organe d'entraînement (24, 24'), et des portions souples (44) entre deux pions d'accrochage (45) voisins, les portions souples étant solidaires des pions d'accrochage, chaque organe d'entraînement (24, 24') étant attaché à un pion d'accrochage (45),
**caractérisé en ce que**
les surfaces d'entraînement (46) des pions d'accrochage étant destinées à venir en contact avec l'organe médical souple allongé.

2. Module robotisé selon la revendication 1, dans lequel la paire (33) d'organes d'entraînement (24, 24') est plaçable alternativement dans la configuration d'entraînement et dans une configuration libre dans laquelle la surface d'entraînement (34, 34') des organes d'entraînement (24, 24') de la paire (33) d'organes d'entraînement (24, 24') n'est pas en prise avec l'organe médical souple allongé,
dans lequel l'organe de commande (18, 11) est adapté pour commander de manière répétée cyclique le déplacement par rapport à la base (132) des organes d'entraînement (24, 24') en configuration d'entraînement de la première à la deuxième position, et un déplacement par rapport à la base (132) des organes d'entraînement (24, 24') de la paire (33) d'organes d'entraînement (24, 24') en configuration libre de la deuxième à la première position sans entraîner l'organe médical souple allongé par rapport à la base.

3. Module robotisé d'entraînement selon la revendication 2, dans lequel la base (132) est une première base, la paire (33) d'organes d'entraînement (24, 24') est une première paire d'organes d'entraînement, le module robotisé comprenant en outre :
- une deuxième base (132'),
- une deuxième paire (33') d'organes d'entraînement (24", 24‴) présentant chacun une surface d'entraînement (34", 34‴), la deuxième paire (33') d'organes d'entraînement (24", 24‴) étant plaçable dans une configuration d'entraînement dans laquelle les surfaces d'entraînement (34", 34‴) des organes d'entraînement (24", 24‴) de la deuxième paire (33') d'organes d'entraînement (24", 24‴) sont en prise avec l'organe médical souple allongé à entraîner et disposés de part et d'autre de celui-ci,
la deuxième paire (33') d'organes d'entraînement (24", 24‴) étant montée mobile par rapport à la deuxième base (132') selon un degré de liberté entre une première et une deuxième positions,
- l'organe de commande (18, 11) étant adapté en outre pour commander un déplacement par rapport à la base (132') des organes d'entraînement (24", 24‴) de la deuxième paire (33') d'organes d'entraînement (24", 24‴) en configuration d'entraînement de la première à la deuxième position, entraînant ainsi l'organe médical souple allongé par rapport à la deuxième base (132').

4. Module robotisé selon la revendication 3, comprenant en outre l'une et/ou l'autre des dispositions suivantes :
- la deuxième paire (33) d'organes d'entraînement (24", 24‴) est plaçable alternativement dans la configuration d'entraînement et dans une configuration libre dans laquelle la surface d'entraînement (34", 34‴) des organes d'entraînement (24", 24‴) de la deuxième paire (33') d'organes d'entraînement (24", 24‴) n'est pas en prise avec l'organe médical souple allongé, et l'organe de commande (18, 11) est adapté pour commander de manière répétée cyclique le déplacement par rapport à la deuxième base (132') des organes d'entraînement (24", 24‴) en configuration d'entraînement de la première à la deuxième position, et un déplacement par rapport à la deuxième base (132') des organes d'entraînement (24", 24‴) de la deuxième paire (33') d'organes d'entraînement (24", 24‴) en configuration libre de la deuxième à la première position sans entraîner l'organe médical souple allongé par rapport à la deuxième base ;
- la première base (132) et la deuxième base (132') sont solidaires ou communes ;
- la barrière stérile (39) consommable à usage unique comprend un pion d'accrochage (45) pour chaque organe d'entraînement (24'', 24''') de la deuxième paire (33'), et des portions souples (44) entre deux pions d'accrochage (45) voisins, les portions souples étant solidaires des pions d'accrochage, chaque organe d'entraînement (24'', 24''') de la deuxième paire étant attaché à un pion d'accrochage (45), les surfaces d'entraînement (46) des pions d'accrochage étant destinées à venir en contact avec l'organe médical souple allongé.

5. Module robotisé d'entraînement selon l'une quelconque des revendications 1 à 4, comprenant en outre l'une et/ou l'autre des dispositions suivantes :
- la barrière stérile comprend un film (42) continu consommable à usage unique comprenant une portion d'attachement (43) pour chaque organe d'entraînement (24, 24'), et des portions souples (44) entre deux portions d'attachement (43) voisines, chaque organe d'entraînement (24, 24') étant attaché à une portion d'attachement (43) du film (42) continu, les surfaces d'entraînement (34, 34') étant en prise avec l'organe médical souple allongé par l'intermédiaire du film (42) continu ;
- la barrière stérile (39) comprend également un carter (60) rigide, chaque pion d'accrochage (45) étant assemblé au carter (60) rigide par l'intermédiaire desdites portions souples (44) ;
- le carter (60) rigide comprend une pluralité de fenêtres (61) fermées chacune de manière étanche par une portion souple (44) ;
- le pion d'accrochage (45) est assemblé à l'organe d'entraînement (24, 24') par l'intermédiaire de zones frangibles reliant la surface d'entraînement (46), en contact avec l'organe médical souple allongé, à la portion d'assemblage (54) à l'organe d'entraînement ;
- la barrière stérile (39) sépare l'espace en deux sous-espaces : un sous-espace stérile comprenant l'organe médical souple allongé, et un sous-espace pas nécessairement stérile dans lequel sont disposés les organes d'entraînement (24, 24').

6. Module robotisé d'entraînement selon l'une quelconque des revendications 1 à 5, comprenant un boîtier (35) fermé englobant les organes d'entraînement (24, 24') et, le cas échéant, la base (132), et comportant une lumière (36) amont et une lumière (36) aval à travers lesquelles s'étend l'organe médical souple allongé.

7. Module robotisé d'entraînement selon la revendication 6, dans lequel le boîtier (35) comprend également un organe de liaison (27) de tout organe d'entraînement (24, 24') à un moteur externe au boîtier.

8. Module robotisé d'entraînement selon la revendication 6 ou 7, dans lequel le boîtier (35) comporte un réceptacle (47) et un couvercle (48) mobile par rapport au réceptacle (47) entre une configuration fermée où un accès par un opérateur à l'organe médical souple allongé à l'intérieur du boîtier (35) est interdit, et une configuration ouverte où un accès par un opérateur à l'organe médical souple allongé à l'intérieur du boîtier (35) est possible ;
et notamment dans lequel une portion finale de la course du couvercle (48) depuis sa configuration ouverte à sa configuration fermée est un mouvement de translation, et/ou dans lequel, en configuration fermée, au voisinage des organes d'entraînement (24, 24'), un volume libre important est disponible entre le couvercle (48) et le réceptacle (47), et en dehors de ce voisinage, un volume libre réduit est disponible entre le couvercle (48) et le réceptacle (47), y permettant un guidage de l'organe médical souple allongé.

9. Module robotisé selon l'une des revendications 6 à 8, dans lequel le boîtier (35) comporte une portion de base à demeure (101) portant les organes d'entraînement (24, 24'), et une cassette (102) consommable à usage unique assemblable/désassemblable de la portion de base, la cassette comprenant au moins la barrière stérile (39).

10. Module robotisé selon l'une quelconque des revendications 1 à 9, dans lequel chaque pion d'accrochage (45) est détachable d'un organe d'entraînement (24, 24') respectif.

11. Module robotisé selon l'une quelconque des revendications 1 à 10, comprenant en outre un véhicule (87) porteur de pions d'accrochage, rigide, chaque pion d'accrochage (45) étant assemblé de manière provisoire au véhicule (87).

12. Module robotisé selon la revendication 11, comprenant en outre l'une et/ou l'autre des dispositions suivantes :
- le véhicule (87) comprend en outre une portion de détrompage (80) empêchant son assemblage selon une orientation non souhaitée ;
- le véhicule (87) est désassemblable de la barrière stérile (39).

13. Module robotisé selon l'une quelconque des revendications 1 à 12, dans lequel chaque pion d'accrochage (45) comporte une paroi plane (62) comportant une face avant, et comprenant un système barrière (98) de limitation du déplacement de l'organe médical souple allongé, maintenant l'organe médical souple allongé en regard de la face avant ;
Et notamment dans lequel chaque barrière du système barrière (98) de limitation comprend une alternance de creux (99) et de saillies (100).

14. Robot d'artériographie comprenant un récipient (14), un organe médical souple allongé (15, 15', 15'') au moins partiellement contenu dans le récipient (14), un module robotisé (16) d'entraînement selon l'une des revendications 1 à 13 fixé au récipient (14), et adapté pour l'entraînement de l'organe médical souple allongé (15, 15', 15") hors du récipient (14).

15. Cassette pour un module robotisé d'entraînement d'organe médical souple allongé comprenant une barrière stérile (39) comprenant des pions d'accrochage (45) adaptés pour être attachés chacun à un organe d'entraînement (24, 24') d'un organe médical souple allongé, et des portions souples (44) entre deux pions d'accrochage (45) voisins, les portions souples étant solidaires des pions d'accrochage,
**caractérisé en ce que** les surfaces d'entraînement (46) des pions d'accrochage étant destinées à venir en contact avec l'organe médical souple allongé de manière à pouvoir entraîner cet organe médical souple allongé.

## Patentansprüche

1. Robotermodul zum Antrieb eines länglichen flexiblen medizinischen Elements, umfassend:
- eine Basis (132),
- ein Paar (33) von Antriebselementen (24, 24'), die jeweils eine Antriebsfläche (34, 34') aufweisen, wobei das Paar (33) von Antriebselementen (24, 24') in einer Antriebskonfiguration platzierbar ist, in der die Antriebsflächen (34, 34') der Antriebselemente (24, 24') des Paars (33) von Antriebselementen (24, 24') mit dem anzutreibenden flexiblen, länglichen medizinischen Element in Eingriff stehen und auf beiden Seiten desselben angeordnet sind,
wobei das Paar (33) von Antriebselementen (24, 24') in Bezug auf die Basis (132) mit einem Freiheitsgrad zwischen einer ersten und einer zweiten Position beweglich montiert ist,
- ein Steuerelement (18, 11), das dazu ausgebildet ist, eine Bewegung relativ zur Basis (132) der Antriebselemente (24, 24') des Paars (33) von Antriebselementen (24, 24') in der Antriebskonfiguration von der ersten in die zweite Position zu steuern, wodurch das flexible längliche medizinische Element relativ zur Basis (132) angetrieben wird,
- eine sterile, verbrauchbare Einwegbarriere (39), die einen Haltestift (45) für jedes Antriebselement (24, 24') und flexible Abschnitte (44) zwischen zwei benachbarten Haltestiften (45) umfasst, wobei die flexiblen Abschnitte mit den Haltestiften fest verbunden sind und jedes Antriebselement (24, 24') an einem Haltestift (45) befestigt ist,
**dadurch gekennzeichnet, dass** die Antriebsflächen (46) der Haltestifte dazu bestimmt sind, mit dem länglichen flexiblen medizinischen Element in Kontakt zu kommen.

2. Robotermodul nach Anspruch 1, wobei das Paar (33) von Antriebselementen (24, 24') alternativ in der Antriebskonfiguration und in einer freien Konfiguration platzierbar ist, in der die Antriebsfläche (34, 34') der Antriebselemente (24, 24') des Paares (33) von Antriebselementen (24, 24') nicht mit dem länglichen flexiblen medizinischen Element in Eingriff steht,
wobei das Steuerelement (18, 11) dazu ausgebildet ist, zyklisch wiederholt die Bewegung relativ zur Basis (132) der Antriebselemente (24, 24') in der Antriebskonfiguration von der ersten in die zweite Position zu steuern, und eine Bewegung relativ zur Basis (132) der Antriebselemente (24, 24') des Paars (33) von Antriebselementen (24, 24') in freier Konfiguration von der zweiten zur ersten Position, ohne das flexible längliche medizinische Element relativ zur Basis mitzunehmen.

3. Roboterantriebsmodul nach Anspruch 2, wobei die Basis (132) eine erste Basis ist, das Paar (33) von Antriebselementen (24, 24') ein erstes Paar von Antriebselementen ist, wobei das Robotermodul ferner umfasst:
- eine zweite Basis (132'),
- ein zweites Paar (33') von Antriebselementen (24", 24‴), die jeweils eine Antriebsfläche (34", 34‴) aufweisen, wobei das zweite Paar (33') von Antriebselementen (24", 24‴) in einer Antriebskonfiguration platzierbar ist, in der die Antriebsflächen (34", 34‴) der Antriebselemente (24", 24‴) des zweiten Paars (33') von Antriebselementen (24", 24‴) mit dem anzutreibenden länglichen flexiblen medizinischen Element in Eingriff stehen und auf beiden Seiten desselben angeordnet sind,
wobei das zweite Paar (33') von Antriebselementen (24", 24‴) in Bezug auf die zweite Basis (132') mit einem Freiheitsgrad zwischen einer ersten und einer zweiten Position beweglich montiert ist,
- wobei das Steuerelement (18, 11) ferner dazu ausgebildet ist, eine Bewegung der Antriebselemente (24", 24‴) des zweiten Paars (33') von Antriebselementen (24", 24‴) relativ zur Basis (132') in Antriebskonfiguration von der ersten in die zweite Position zu steuern, wodurch das flexible längliche medizinische Element relativ zur zweiten Basis (132') angetrieben wird.

4. Robotermodul nach Anspruch 3, ferner umfassend die eine und/oder die andere der folgenden Bestimmungen:
- das zweite Paar (33) von Antriebselementen (24", 24‴) kann alternativ in der Antriebskonfiguration und in einer freien Konfiguration platzierbar sind, in der die Antriebsfläche (34", 34‴) der Antriebselemente (24", 24‴) des zweiten Paars (33') von Antriebselementen (24", 24‴) nicht mit dem länglichen flexiblen medizinischen Element in Eingriff steht, und das Steuerelement (18, 11) ist dazu ausgebildet, zyklisch wiederholt die Bewegung relativ zur zweiten Basis (132') der Antriebselemente (24", 24‴) in der Antriebskonfiguration von der ersten zur zweiten Position zu steuern und eine Bewegung relativ zur zweiten Basis (132') der Antriebselemente (24", 24‴) des zweiten Paars (33') von Antriebselementen (24", 24‴) in freier Konfiguration von der zweiten in die erste Position zu steuern, ohne das flexible längliche medizinische Element in Bezug auf die zweite Basis anzutreiben;
- die erste Basis (132) und die zweite Basis (132') sind fest miteinander verbunden oder eins;
- die sterile, verbrauchbare Einwegbarriere (39) umfasst einen Haltestift (45) für jedes Antriebselement (24", 24‴) des zweiten Paares (33') und flexible Abschnitte (44) zwischen zwei benachbarten Haltestift (45), wobei die flexiblen Abschnitte mit den Haltestiften fest verbunden sind, wobei jedes Antriebselement (24" ; 24‴) des zweiten Paares an einem Haltestift (45) befestigt ist, wobei die Antriebsflächen (46) der Haltestifte dazu bestimmt sind, mit dem länglichen flexiblen medizinischen Element in Kontakt zu kommen.

5. Roboterantriebsmodul nach einem der Ansprüche 1 bis 4, ferner umfassend die eine und/oder die andere der folgenden Bestimmungen:
- die sterile Barriere umfasst eine verbrauchbare durchgehende Einwegfolie (42) mit einem Befestigungsabschnitt (43) für jedes Antriebselement (24, 24') und flexiblen Abschnitten (44) zwischen zwei benachbarten Befestigungsabschnitten (43), wobei jedes Antriebselement (24, 24') an einem Befestigungsabschnitt (43) der durchgehenden Folie (42) befestigt ist, wobei die Antriebsflächen (34, 34') über die durchgehende Folie (42) mit dem länglichen flexiblen medizinischen Element in Eingriff stehen;
- die sterile Barriere (39) umfasst auch ein starres Gehäuse (60), wobei jeder Haltestift (45) über die flexiblen Abschnitte (44) mit dem starren Gehäuse (60) verbunden ist;
- das starre Gehäuse (60) umfasst eine Vielzahl von Fenstern (61), die jeweils durch einen flexiblen Abschnitt (44) abgedichtet sind;
- der Haltestift (45) ist mit dem Antriebselement (24, 24') über zerbrechbare Bereiche verbunden, die die Antriebsfläche (46), die mit dem länglichen flexiblen medizinischen Element in Kontakt steht, mit dem Verbindungsabschnitt (54) des Antriebselements verbinden;
- die sterile Barriere (39) teilt den Raum in zwei Unterräume: einen sterilen Unterraum, der das flexible längliche medizinische Element umfasst, und einen nicht notwendigerweise sterilen Unterraum, in dem die Antriebselemente (24, 24') angeordnet sind.

6. Roboterantriebsmodul nach einem der Ansprüche 1 bis 5, umfassend ein festes Gehäuse (35), das die Antriebselemente (24, 24') und gegebenenfalls die Basis (132) umschließt und ein stromaufwärts gelegenes Licht (36) und ein stromabwärts gelegenes Licht (36) umfasst, durch die sich das flexible längliche medizinische Element erstreckt.

7. Roboterantriebsmodul nach Anspruch 6, wobei das Gehäuse (35) auch ein Element zur Verbindung (27) jedes Antriebselements (24, 24') mit einem Motor außerhalb des Gehäuses umfasst.

8. Roboterantriebsmodul nach Anspruch 6 oder 7, wobei das Gehäuse (35) eine Aufnahme (47) und einen Deckel (48) umfasst, der in Bezug auf die Aufnahme (47) zwischen einer geschlossenen Konfiguration, in der ein Zugang durch einen Bediener zu dem länglichen flexiblen medizinischen Element im Inneren des Gehäuses (35) verboten ist, und einer offenen Konfiguration, in der ein Zugang durch einen Bediener zu dem länglichen flexiblen medizinischen Element im Inneren des Gehäuses (35) möglich ist, beweglich ist;
und wobei insbesondere ein Endabschnitt des Hubs des Deckels (48) von seiner offenen zu seiner geschlossenen Konfiguration eine Translationsbewegung ist, und/oder wobei in der geschlossenen Konfiguration in der Nähe der Antriebselemente (24, 24') ein großes freies Volumen zwischen dem Deckel (48) und dem Behälter (47) verfügbar ist, und außerhalb dieser Nähe ein reduziertes freies Volumen zwischen dem Deckel (48) und dem Behälter (47) verfügbar ist, was eine Führung des länglichen flexiblen medizinischen Elements dort ermöglicht.

9. Robotermodul nach einem der Ansprüche 6 bis 8, wobei das Gehäuse (35) einen feststehenden Basisabschnitt (101) umfasst, der die Antriebselemente (24, 24') trägt, und eine verbrauchbare Einwegkassette (102) aufweist, die mit dem Basisabschnitt zusammensetzbar/zerlegbar ist, wobei die Kassette wenigstens die sterile Barriere (39) umfasst.

10. Robotermodul nach einem der Ansprüche 1 bis 9, wobei jeder Haltestift (45) von einem jeweiligen Antriebselement (24, 24') abnehmbar ist.

11. Robotermodul nach einem der Ansprüche 1 bis 10, ferner umfassend ein Vehikel (87), das starre Haltestifte trägt, wobei jeder Haltestift (45) provisorisch an dem Vehikel (87) angebracht ist.

12. Robotermodul nach Anspruch 11, ferner umfassend die eine und/oder die andere der folgenden Bestimmungen:
- das Vehikel (87) umfasst ferner einen Kodierungsabschnitt (80), der verhindert, dass es in einer unerwünschten Orientierung zusammengebaut wird;
- das Vehikel (87) ist von der sterilen Barriere (39) abnehmbar.

13. Robotermodul nach einem der Ansprüche 1 bis 12, wobei jeder Haltestift (45) eine ebene Wand (62) mit einer Vorderseite aufweist und ein Barrieresystem (98) zur Begrenzung der Bewegung des länglichen flexiblen medizinischen Elements umfasst, das das flexible längliche medizinische Element gegenüber der Vorderseite hält;
und insbesondere wobei jede Barriere des Barrierensystems (98) zur Begrenzung eine Abfolge von Vertiefungen (99) und Vorsprüngen (100) umfasst.

14. Arteriographieroboter, umfassend einen Behälter (14), ein längliches flexibles medizinisches Element (15, 15', 15"), das zumindest teilweise in dem Behälter (14) enthalten ist, ein Roboterantriebsmodul (16) nach Anspruch 1 bis 13, das an dem Behälter (14) befestigt ist und zum Antrieb des länglichen flexiblen medizinischen Elements (15, 15', 15") außerhalb des Behälters (14) ausgebildet ist.

15. Kassette für ein Robotermodul zum Antrieb eines länglichen medizinischen Elements, umfassend eine sterile Barriere (39), umfassend Haltestifte (45), die dazu ausgebildet sind, jeweils an einem Antriebselement (24, 24') eines länglichen flexiblen medizinischen Elements befestigt zu werden, und flexible Abschnitte (44) zwischen zwei benachbarten Haltestiften (45), wobei die flexiblen Abschnitte mit den Haltestiften fest verbunden sind, **dadurch gekennzeichnet, dass** die Antriebsflächen (46) der Haltestifte dazu bestimmt sind, in Kontakt mit dem flexiblen länglichen medizinischen Element zu kommen, so dass sie dieses flexible längliche medizinische Element antreiben können.

## Claims

1. Robotic drive module for an elongate flexible medical device comprising:
- a base (132),
- a pair (33) of drive members (24, 24') each having a drive surface (34, 34'), the pair (33) of drive members (24, 24') being placeable in a drive configuration in which the drive surfaces (34, 34') of the drive members (24, 24') of the pair (33) of drive members (24, 24') engage with the elongate flexible medical device to be driven, and are arranged on either side thereof,
the pair (33) of drive members (24, 24') being movably mounted relative to the base (132) along one degree of freedom between a first and second position,
- a control member (18, 11) suitable for controlling a movement relative to the base (132) of the drive members (24, 24') of the pair (33) of drive members (24, 24') in the drive configuration from the first to the second position, thus driving the elongate flexible medical device relative to the base (132),
- a disposable consumable sterile barrier (39) comprising an attachment bracket (45) for each drive member (24, 24'), and flexible portions (44) between two adjacent attachment brackets (45), the flexible portions being connected to the attachment brackets, each drive member (24, 24') being attached to an attachment bracket (45), **characterised in that** the drive surfaces (46) of the attachment brackets are intended to come into contact with the elongate flexible medical device.

2. Robotic module according to claim 1, wherein the pair (33) of drive members (24, 24') can be placed alternately in the drive configuration and in a free configuration in which the drive surface (34, 34') of the drive members (24, 24') of the pair (33) of drive members (24, 24') is not engaged with the elongate flexible medical device, wherein the control member (18, 11) is suitable for controlling, in a repeated cyclical manner, the movement of the drive members (24, 24') relative to the base (132), in the drive configuration, from the first to the second position, and a movement relative to the base (132) of the drive members (24, 24') of the pair (33) of drive members (24, 24'), in free configuration, from the second to the first position without driving the elongate flexible medical device relative to the base.

3. Robotic module according to claim 2, wherein the base (132) is a first base, the pair (33) of drive members (24, 24') is a first pair of drive members, the robotic module further comprising:
- a second base (132'),
- a second pair (33') of drive members (24", 24‴) each having a drive surface (34", 34‴), the second pair (33') of drive members (24", 24‴) being placeable in a drive configuration in which the drive surfaces (34", 34"') of the drive members (24", 24‴) of the second pair (33') of drive members (24", 24‴) engage with the elongate flexible medical device to be driven and are arranged on either side thereof, the second pair (33') of drive members (24", 24‴) being movably mounted relative to the second base (132') along one degree of freedom between a first and second position,
- the control member (18, 11) being suitable for further controlling a movement relative to the base (132') of the drive members (24", 24‴) of the second pair (33') of drive members (24", 24‴), in the drive configuration, from the first to the second position, thus driving the elongate flexible medical device relative to the second base (132').

4. Robotic module according to claim 3, further comprising either one or both of the following arrangements:
- the second pair (33) of drive members (24", 24‴) can be placed alternately in the drive configuration and in a free configuration in which the drive surface (34", 34‴) of the drive members (24", 24‴) of the second pair (33') of drive members (24", 24‴) is not engaged with the elongate flexible medical device, and the control member (18, 11) is suitable for controlling, in a repeated cyclical manner, the movement of the drive members (24", 24"') relative to the second base (132'), in the drive configuration, from the first to the second position, and a movement relative to the second base (132') of the drive members (24", 24"') of the second pair (33') of drive members (24", 24‴), in free configuration, from the second to the first position without driving the elongate flexible medical device relative to the base;
- the first base (132) and the second base (132') are connected together or common;
- the disposable consumable sterile barrier (39) comprises an attachment bracket (45) for each drive member (24", 24‴) of the second pair (33'), and flexible portions (44) between two adjacent attachment brackets (45), the flexible portions being connected to the attachment brackets, each drive member (24", 24‴) of the second pair being attached to an attachment bracket (45), the drive surfaces (46) of the attachment brackets being intended to come into contact with the elongate flexible medical device.

5. Robotic drive module according to any one of claims 1 to 6, further comprising either one or both of the following arrangements:
- the sterile barrier comprises a disposable continuous consumable film (42) comprising an attachment portion (43) for each drive member (24, 24'), and flexible portions (44) between two adjacent attachment portions (43), each drive member (24, 24') being attached to an attachment portion (43) of the continuous film (42), the drive surface (34, 34') being engaged with the elongate flexible medical device by means of the continuous film (42);
- the sterile barrier (39) also comprises a rigid casing (60), each attachment bracket (45) being assembled on the rigid casing (60) by means of said flexible portions (44);
- the rigid casing (60) comprises a plurality of windows (61) each sealingly closed by a flexible portion (44);
- the attachment bracket (45) is assembled on the drive member (24, 24') by means of frangible zones connecting the drive surface (46), in contact with the elongate flexible medical device, to the assembly portion (54) on the drive member;
- the sterile barrier (39) separates the space into two sub-spaces: a sterile sub-space containing the elongate flexible medical device, and a sub-space that is not necessarily sterile in which the drive members (24, 24') are disposed.

6. Robotic drive module according to any one of claims 1 to 5, comprising a closed housing (35) encompassing the drive members (24, 24') and, where applicable, the base (132), and comprising an upstream port (36) and a downstream port (36) through which the elongate flexible medical device extends.

7. Robotic drive module according to claim 6, wherein the housing (35) also comprises a member (27) for connecting any drive member (24, 24') to a motor external to the housing.

8. Robotic drive module according to claim 6 or 7, wherein the housing (35) comprises a receptacle (47) and a lid (48) movable relative to the receptacle (47) between a closed configuration, where access by an operator to the elongate flexible medical device inside the housing (35) is prohibited, and an open configuration where access by an operator to the elongate flexible medical device inside the housing (35) is possible;
and in particular wherein a final portion of the trajectory of the lid (48) from its open configuration to its closed configuration is a translational movement,
and wherein, in closed configuration, in the vicinity of the drive members (24, 24'), a large free volume is available between the lid (48) and the receptacle (47), and outside of this vicinity, a reduced free volume is available between the lid (48) and the receptacle (47), allowing guidance therein of the elongate flexible medical device.

9. Robotic module according to one of claims 6 to 8, wherein the housing (35) comprises a remote base portion (101) bearing the drive members (24, 24'), and a disposable consumable cassette (102), that can be assembled on and disassembled from the base portion, the cassette comprising at least the sterile barrier (39).

10. Robotic module according to any one of claims 1 to 9, wherein each attachment bracket (45) can be detached from a respective drive member (24, 24').

11. Robotic module according to any one of claims 1 to 10, further comprising a rigid attachment-bracket-bearing carrier (87), each attachment bracket (45) being temporarily assembled on the carrier (87).

12. Robotic module according to claim 11, further comprising either one or both of the following arrangements:
- the carrier (87) further comprises a foolproof portion (80) preventing assembly thereof in an undesired orientation;
- the carrier (87) can be disassembled from the sterile barrier (39).

13. Robotic module according to any one of claims 1 to 12, wherein each attachment bracket (45) comprises a flat wall (62) having a front face, and comprising a barrier system (98) for limiting movement of the elongate flexible medical device, holding the elongate flexible medical device facing the front face;
And in particular wherein each barrier of the limiting barrier system (98) comprises alternate recesses (99) and protrusions (100).

14. Arteriography robot comprising a container (14), an elongate flexible medical device (15, 15', 15") at least partially contained in the container (14), a robotic drive module (16) according to one of claims 1 to 13 attached to the container (14), and suitable for driving the elongate flexible medical device (15, 15', 15") outside the container (14).

15. Cassette for a robotic drive module for an elongate flexible medical device comprising a sterile barrier (39), comprising attachment brackets (45) each capable of being attached to a drive member (24, 24') of an elongate flexible medical device, and flexible portions (44) between two adjacent attachment brackets (45), the flexible portions being connected to the attachment brackets,
**characterised in that** the drive surfaces (46) of the attachment brackets are intended to come into contact with the elongate flexible medical device so as to be able to drive this elongate flexible medical device.
